# EUROPÄISCHE PATENTSCHRIFT

(11) **EP 2 109 492 B1**
(45) Veröffentlichungstag und Bekanntmachung des Hinweises auf die Patenterteilung: **31.10.2012**
(21) Anmeldenummer: 08701002.1
(22) Anmeldetag: 08.01.2008
(51) Int. Cl.: B01D 53/22, B01D 63/02, B01D 69/02, B01D 69/08, B01D 71/70, A61M 1/16, B29C 44/34, C08J 9/04

(54) **GESCHÄUMTE MEMBRAN INSBESONDERE ZUM EINSATZ IN EINER KÜNSTLICHEN LUNGE SOWIE HERSTELLUNGSVERFAHREN HIERFÜR**
GAS EXCHANGE MEMBRANE, PARTICULARLY FOR USE IN AN ARTIFICIAL LUNG, AND METHOD FOR THE PRODUCTION OF SUCH A GAS EXCHANGE MEMBRANE
MEMBRANE D'ÉCHANGE GAZEUX À UTILISER NOTAMMENT DANS UN POUMON ARTIFICIEL ET PROCÉDÉ DE PRODUCTION D'UNE TELLE MEMBRANE D'ÉCHANGE GAZEUX

(30) Priorität: 11.01.2007 DE 102007001665
(43) Veröffentlichungstag der Anmeldung: 21.10.2009
(73) Patentinhaber: RAUMEDIC AG, 95213 Münchberg (DE)
(72) Erfinder: ZIEMBINSKI, Ralf, 95189 Köditz-Brunnenthal (DE)
(74) Vertreter: Hofmann, Matthias
(86) Internationale Anmeldenummer: PCT/EP2008/000057
(87) Internationale Veröffentlichungsnummer: WO 2008/083943

(56) Entgegenhaltungen:
- EP-A- 0 754 488
- EP-A- 0 803 259
- WO-A-00/26006
- WO-A-02/04554
- WO-A-99/38604
- DE-A1- 10 012 308
- JP-A- 2004 050 042

## Beschreibung

Die Erfmdung betrifft eine Gasaustauschmembranvorrichtung für eine medizinische Anwendung, insbesondere zum Einsatz in einer künstlichen Lunge. Ferner betrifft die Erfmdung ein Verfahren zur Herstellung einer derartigen Gasaustauschmembranvorrichtung.

Derartige Gasaustauschmedien sind beispielsweise aus der EP 0 765 683 B1, der EP 0 803 259 A1, der DE 42 38 884 A1, der DE 600 03 112 T2 und der DE 100 34 098 A1 bekannt. Die Gasaustauschmembran wird in einem Oxygenator verwendet, um Blut bzw. Blutplasma Sauerstoff zuzuführen und aus dem Blut bzw. Blutplasma durch Stoffwechselprozesse entstehendes Kohlendioxid abzuführen. Die Gasaustauschmembran ist insbesondere in Form einer Hohlfasermembran bekannt. Die Hohlfasern sind bei den bekannten Oxygenatoren z. B. aus Polypropylen (PP) oder aus Polymethylpenten (PMP) gefertigt. Beide Werkstoffe gehören zur Polymerklasse der Polyolefine und haben keine gute Blutverträglichkeit. Bei diesen Werkstoffen kann eine Aktivierung des Blutgerinnungssystems, vor allem der Thrombozyten, beobachtet werden. Zudem kann es durch Fehlstellen bzw. Leckagen der bekannten Hohlfasern zu einem Austritt von Blut bzw. Blutplasma aus dem Blutkreislauf des Oxygenators kommen. Blut bzw. Blutplasma gelangt dann unerwünscht auf die nicht sterile Seite der Gasaustauschmembran. Zudem sind die bekannten Hohlfasermembranen in ihrer Gasaustauschleistung beschränkt, so dass die bekannten Oxygenatoren recht voluminös sind. Das große Volumen der bekannten Oxygenatoren hat wiederum den Nachteil, dass eine erhebliche Menge Blut bzw. Blutplasma für den Gasaustausch bereit gestellt werden muss. Gasaustauschmembranen kommen auch bei anderen medizinischen Anwendungen zum Einsatz, z. B. als Auflagen bei Verbrennungen.

Die EP 0 754 488 A1 beschreibt Gastrennmembranen, unter anderem für medizinische Anwendungen.

Die JP 2004/050042 A beschreibt ein Membranmodul aus einem Silikonschaum zur Gastrennung.

Die DE 100 12 308 A1 beschreibt Hohlfasermembranen, die sowohl zum Gasaustausch als auch in Trennverfahren zum Einsatz kommen können. Die Hohlfasern werden gesponnen. Auf eine Porenerzeugung durch Zusatz von Treibmitteln ist hingewiesen.

Die WO 99/38604 A1 diskutiert mehrere Druckschriften, die sich mit der Herstellung offenporiger und geschlossenporiger Membranen beschäftigen.

Die WO 02/04554 A 1 beschreibt eine gas- bzw. flüssigkeitsdurchlässige bikontinuierliche Schaumstruktur.

Die WO 00/26006 A1 beschreibt ein Verfahren zur Herstellung mikrozellularer Schäume für die Ausbildung von Formkörpern.

Es ist eine Aufgabe der vorliegenden Erfmdung, eine Gasaustauschmembran der eingangs genannten Art derart weiter zu bilden, dass deren Gasaustauschleistung erhöht ist.

Diese Aufgabe ist erfindungsgemäß gelöst durch eine Gasaustauschmembranvorrichtung mit den in Anspruch 1 angegebenen Merkmalen.

Das erfindungsgemäße geschlossenporige geschäumte Material bietet eine gute Trennung zwischen der Gas- und der Blutseite der Gasaustauschmembran, so dass es nicht zu einem unerwünschten Übertritt von Blut bzw. Blutplasma auf die Gasseite kommen kann. Zudem lassen sich aus geschäumtem Material Gasaustauschinedien mit einer Gasaustauschleistung herstellen, die derjenigen der bekannten Hohlfasern deutlich überlegen ist. Die erhöhte Gasaustauschleistung ist auf die deutlich höhere Gaspermeabilität des verwendeten Werkstoffs der erfindungsgemäßen Gasaustauschmembran zurückzuführen. Dies führt zur Möglichkeit der Entwicklung von kompakten Oxygenatoren, für die im Vergleich zum Stand der Technik nur ein geringeres Blutvolumen bereit gestellt werden muss. Dies verringert den Blutbedarf im Rahmen einer Operation erheblich. Typische Gasaustauschflächen liegen bei 1 bis 2 m². Erfindungsgemäß wurde erkannt, dass sich aus geschäumten vernetztem Silikon Kautschuk, eine Gasaustauschmembran herstellen lässt, welche auf Grund der inerten Eigenschaften des Silikonmaterials gut blutverträglich ist. Daher ist die Aktivierung des Blutgerinnungssystems bei einer Gasaustauschmembran aus geschäumtem Silikon nicht so stark ausgeprägt, wie bei herkömmlichen, nicht geschäumten Membranen aus PP oder PMP. Die Gaspermeabilität von Silikonkautschuk ist um mehr als einen Faktor 100 größer als die Gasaustauschleistung von Polyolefinen, wie etwa Polyethylen (PE) oder Polypropylen (PP). Der Einsatz von gerinnungshemmenden Wirkstoffen ist bei Verwendung einer erfindungsgemäßen Silikon-Gasaustauschmembran nicht mehr in den bisher üblichen Umfang erforderlich. Insbesondere ist durch das aufgeschäumte Material ein selbsttragender Aufbau der Gasaustauschmembran möglich, bei dem die Gasaustauschmembran also nicht zusätzlich stabilisiert werden muss. Durch das Aufschäumen lassen sich Membranen herstellen, die gleichzeitig sehr geringe Wandstärken und eine hohe Eigenstabilität aufweisen. Insbesondere geschäumte Schläuche sind im Vergleich zu ungeschäumten Schläuchen besser verarbeitbar, da bei den geschäumten Gasaustauschmembranen eine Gefahr des Kollabierens nicht vorliegt. Die erfmdungsgemäße Gasaustauschmembran kann nicht nur in einer künstlichen Lunge, sondern auch bei anderen medizinischen Anwendungen zum Einsatz kommen. Ein Beispiel hierfür ist die Verwendung der Gasaustauschmembran als Auflage zur Behandlung von Verbrennungen. In diesem Fall stellt die Auflage sicher, dass einerseits das durch die Verbrennung geschädigte Gewebe insbesondere gegen das Eindringen von Verunreinigungen geschützt ist, wobei andererseits ein zur Wundheilung notwendiger Gasaustausch erfolgen kann. Die schlauchförmige Gasaustauschmembran bietet die Möglichkeit eines besonders kompakten Aufbaus eines hiermit ausgerüsteten Oxygenators. Dies gilt insbesondere für das Schlauchbündel.

Platinvernetzter Schaum nach Anspruch 2 hat sich zum Einsatz in einer Gasaustauschmembran als besonders geeignet herausgestellt. Bei einer Gasaustauschmembran aus geschäumtem, geschlossenporigem Silikonkautschuk führt die Vernetzung zur Fixierung der kaltgeformten, insbesondere extrudierten Form. Alternativ zu einer Platinvernetzung kann auch eine peroxidische Vernetzung stattfinden.

Dimensionen des Schlauchs und der Schaumkavitäten nach den Ansprüchen 3 bis 5 haben sich zur Schaffung einer hohen Gasdurchlässigkeit bei gleichzeitig guter Dichtwirkung zwischen Gas- und Blutseite der Membran als besonders geeignet herausgestellt.

Eine weitere Aufgabe der Erfindung ist es, ein Verfahren zur Herstellung einer erfindungsgemäßen Gasaustauschmembranvorrichtung anzugeben.

Diese Aufgabe ist erfindungsgemäß gelöst durch ein Verfahren mit den in Anspruch 6 angegebenen Merkmalen.

Ein derartiges Verfahren bietet die Möglichkeit, die erfindungsgemäße Gasaustauschmembranvorrichtung mit Massenproduktionstechniken herzustellen. Über den Extrusionsdruck und die Düsenform lässt sich im Zusammenspiel mit den Parametern des verwendeten Treibmittels die typische Kavitätengröße sowie die Verteilung der Kavitätengrößen im Schaum fein beeinflussen. Eingesetzt wird bevorzugt ein Ausgangsmaterial für Silikonelastomere. Der Aufschäumungsprozess erfolgt in der Regel durch Einwirkung von Wärme in einem Vulkanisationsofen. Beim Aufschäumen entstehen aus dem Treibmittel Gase wie CO₂ und/oder Wasserdampf. Eine Schlauchbündelherstellung durch Erzeugen eines schlauchförmigen Extruds, wobei aus dem Extrudat ene Mehrzahl von Schläuchen abgelenkt wird, die anschließend miteinander zu einem Bündel angeordnet und fixiert werden, ist besonders effizient.

Eine Fixierung nach Anspruch 7 ist sicher und blutverträglich.

Ausführungsbeispiele der Erfindung werden nachfolgend an Hand der Zeichnung näher erläutert. In dieser zeigen:
- Fig. 1: schematisch einen Oxygenator als Bestandteil einer künstlichen Lunge;
- Fig. 2: vergrößert ein Gasaustauschmembran des Oxygenators nach Fig. 1 in Form eines Schlauches aus geschäumtem Silikonkautschuk;
- Fig. 3: schematisch eine nicht erfindungsgemäße Ausführung eines Oxygenator;
- Fig. 4: vergrößert das Gasaustauschmembran im Oxygenator nach Fig. 3 in Form einer Platte aus geschäumtem Silikonkautschuk;
- Fig. 5: eine Ausschnittsvergrößerung aus den Figuren 2 und 4 bei einer ersten Ausführung des geschäumten Silikons; und
- Fig. 6: eine Ausschnittsvergrößerung aus den Figuren 2 und 4 bei einer zweiten Ausführung des geschäumten Silikons.

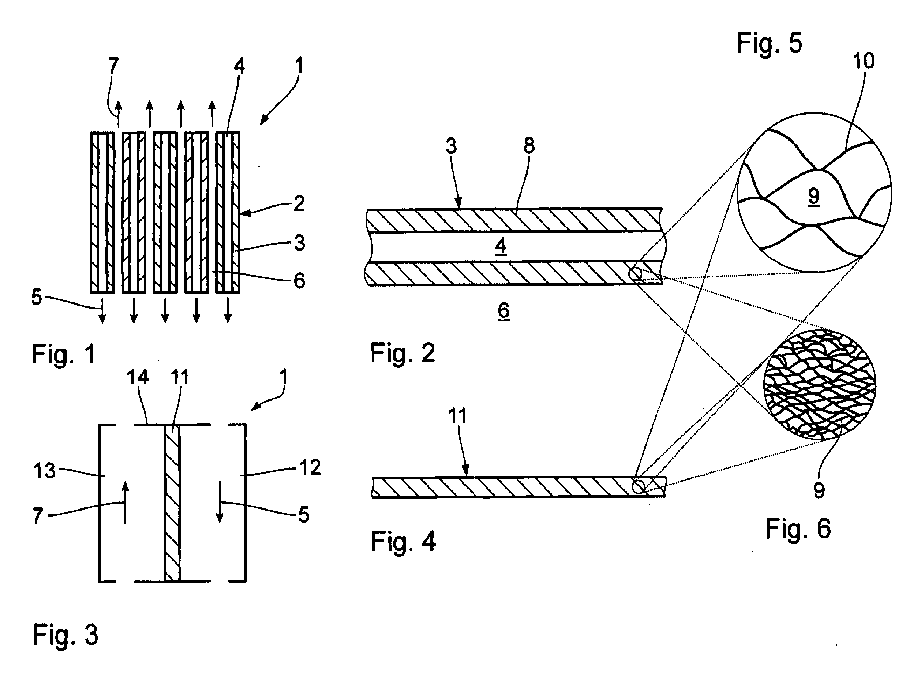

Ein in der Figur 1 schematisch dargestellter Oxygenator 1 dient zur Sauerstoffanreicherung von Blut in einer ansonsten nicht dargestellten künstlichen Lunge einer Herz-Lungen-Maschine. Der prinzipielle Aufbau eines derartigen Oxygenators ist beispielsweise aus der EP 0 765 683 B 1 bekannt. Der Oxygenator 1 ist als Hohlfaser-Membran-Oxygenator aufgebaut und hat ein in einem nicht dargestellten Gehäuse untergebrachtes Bündel 2 von Hohlfasern oder Mikroschläuchen 3, die mit Hilfe eines nicht dargestellten Silikonklebers zueinander fixiert sind. Sauerstoff bzw. ein sauerstoffhaltiges Gas tritt in der Figur 1 von oben in Innenlumen 4 der Schläuche 3 ein und in Fig. 1 unten aus den Innenlumen 4 wieder aus, wie durch Richtungspfeile 5 dargestellt. Blut bzw. Blutplasma tritt von unten in Zwischenräumen 6 zwischen benachbarten Schläuchen 3 in den Oxygenator ein und aus den Zwischenräumen 6 nach oben aus dem Oxygenator 1 wieder aus, wie durch Richtungspfeile 7 verdeutlicht.

Wandungen 8 der Schläuche 3 dienen als Gasaustauschmembranen im Oxygenator 1 zur Zuführung von Sauerstoff zum Blut einerseits und zur Entfernung von durch Stoffwechselprozesse entstehendem Kohlendioxid aus dem Blut andererseits. Weiterhin dienen die Wandungen 8 zur Wärmeübertragung von sauerstoffhaltigem Gas hin zum Blut bzw. Blutplasma, um letzteres auf einer vorgegebenen Temperatur zu halten.

Jeder Schlauch 3 hat einen Außendurchmesser von 0,4 mm. Auch andere Außendurchmesser, die geringer sind als 1,0 mm sind möglich, insbesondere Außendurchmesser, die geringer sind als 0,5 mm. Die Wandstärke der gesamten geschäumten Wandung 8 jedes Schlauchs 3 beträgt 0,1 mm. Auch andere Wandstärken der gesamten geschäumten Wandung 8, die geringer sind als 0,5 mm, sind möglich, bevorzugt Wandstärken, die geringer sind als 0,2 mm.

Jeder Schlauch 3 und damit auch jede Wandung 8 besteht aus platinvernetztem oder peroxidvernetztem geschäumtem Silikon. Kavitäten 9 innerhalb des Schaums (vgl. Fig. 5) haben eine typische Größe zwischen 0,05 und 0,07 mm. Auch andere Kavitätengrößen zwischen 0,01 und 0,1 mm sind möglich. Bevorzugt sind typische Kavitätengrößen zwischen 0,025 und 0,075 mm, noch mehr bevorzugt zwischen 0,05 und 0,07 mm. Fig. 6 zeigt eine Schaumvariante mit einer Kavitätengröße von etwa 0,015 mm.

Der Schaum, aus dem die Wandung 8 der Hohlfasern 3 besteht, ist unabhängig von der Größe der Kavitäten 9 geschlossenporig. Zwischen den Innenlumen 4 und den Zwischenräumen 6 liegt daher mindestens eine geschlossene Mikro- oder Nanowand 10 aus Silikon vor. Bei der Größe der Kavität 9 nach Fig. 5 liegen eine bis vier derartige Mikro/Nanowände 10 zwischen dem Innenlumen 4 und dem Zwischenraum 6 vor. Bei der Größe der Kavität 9 nach Fig. 6 liegen sechs bis 50 derartigen Mikro/Nanowände 10 zwischen dem Innenlumen 4 und dem Zwischenraum 6 vor.

Die Wandstärke der gesamten geschäumten Wandung 8 ist um ein Mehrfaches, meist sogar um ein Vielfaches größer als die Stärke der Mikro/Nanowände 10 zwischen benachbarten Kavitäten 9. Die Mikro/Nanowände 10 haben eine Stärke z. B. im einstelligen Mikrometer-Bereich.

Der Schaum, aus dem die Schläuche 3 bestehen, ist selbsttragend. Es ist daher nicht erforderlich, die Schläuche, zum Beispiel durch zusätzliche Wandungsschichten, zu stabilisieren.

Die Schläuche 3 im Oxygenator 1 werden wie folgt hergestellt: Zunächst wird ein Silikon-Ausgangsmaterial in Schlauchform extrudiert, welches ein Treibmittel enthält. Das Extrudat wird dann bei der Durchleitung durch einen Vulkanisierofen aufgeschäumt. Beim Aufschäumen entstehen aus dem Treibmittel Gase in Form von CO₂ und Wasserdampf. Durch entsprechende Auswahl des Treibmittels kann die Ausbildung toxischer Gase beim Aufschäumen vermieden werden. Aus dem Extrudat wird eine Mehrzahl von Schläuchen 3 abgelängt, die anschließend miteinander zum Bündel 2 angeordnet und mit Hilfe des Silikonklebers fixiert werden. Auch eine andere Art der Fixierung ist möglich, zum Beispiel das Einlegen einer unfixierten Mehrzahl von Schläuchen 3 in ein diese fixierendes Gehäuse.

Die Fig. 3 und 4 zeigen eine nicht erfindungsgemäße Ausführung eines Oxygenators 1 mit einem alternativen Gasaustauschmembran in Form einer Platte 11. Komponenten bzw. Bezugsgrößen, die diejenigen entsprechen, die vorstehend schon unter Bezugnahme auf die Figuren 1, 2, 5 und 6 erläutert wurden, tragen die gleichen Bezugsziffern und werden nicht nochmals im einzelnen diskutiert.

Beim Oxygenator 1 nach Fig. 3 strömt Sauerstoff bzw. ein sauerstoffhaltiges Gas von oben in eine in der Fig. 3 rechte Kammer 12 ein und aus der Kammer 12 nach unten heraus, wie durch den Richtungspfeil 5 verdeutlicht. Blut bzw. Blutplasma strömt in der Fig. 3 von unten in eine in der Fig. 3 linke Kammer 13 ein und nach oben aus der Kammer 13 wieder aus, wie durch den Richtungspfeil 7 verdeutlicht. Die beiden nebeneinander liegenden Kammern 12, 13 sind in einem Gehäuse 14 untergebracht und voneinander durch die Platte 11 getrennt. Die Platte 11 ist wiederum aus platinvernetztem, geschäumtem, geschlossenporigem Silikonkautschuk. Hinsichtlich des Aufbaus und der Kavitätengröße des Schaums, aus dem die Platte 11 gefertigt ist, gilt, was vorstehend zum Schaum der Schläuche 3 erläutert wurde, weswegen die Ausschnittsvergrößerungen nach den Figuren 5 und 6 auch für die Platte 11 gelten. Die Platte hat eine Wandstärke von 0,1 mm. Auch andere Wandstärken im Bereich zwischen 0,05 mm und 2,0 sind möglich, zum Beispiel Wandstärken, die geringer sind als 1,0 mm, bevorzugt geringer sind als 0,5 mm noch mehr bevorzugt geringer als 0,2 mm.

Auch die Platte 11 kann durch Extrusion entsprechend dem hergestellt werden, was vorstehend zur Herstellung der Schläuche 3 erläutert wurde. Die Platte 11 wird dann in das Gehäuse 14 eingeklebt, so dass kein direkter Durchgang zwischen den Kammern 12, 13 möglich ist.

## Patentansprüche

1. Gasaustauschmembranvorrichtung für eine medizinische Anwendung, insbesondere zum Einsatz in einer künstlichen Lunge, umfassend eine Gas austausch membran (3;11) mit einem Aufbau aus geschäumtem, geschlossenporigem Material, wobei die Gasaustauschmembran (3; 11) derart gestaltet ist, dass eine Trennung zwischen einer Gasseite und einer Blutseite der Gasaustauschmembran (3; 11) derart möglich ist, dass ein Gasaustausch durch die Gasaustauschmembran (3; 11) erfolgen kann und dass ein Austausch von flüssigem und/oder festem Medium, insbesondere Blut, Blutplasma und/oder Verunreinigungen, durch die Gasaustauschmembran (3; 11) verhindert ist, wobei das geschäumte Material in Form eines Bündels (2) von Schläuchen (3) ausgebildet ist, wobei die Schläuche (3) miteinander fixiert sind,
**gekennzeichnet durch**
einen Aufbau der Gasaustauschmembranvorrichtung aus geschäumtem, geschlossenporigem, vernetztem Silikonkautschuk.

2. Gasaustauschmembranvorrichtung nach Anspruch 1, **dadurch gekennzeichnet, dass** das geschäumte Material platinvernetzt oder peroxidvernetzt ist.

3. Gasaustauschmembranvorrichtung nach Anspruch 1 oder 2, **dadurch gekennzeichnet, dass** der mindestens eine Schlauch (3) einen Außendurchmesser hat, der geringer ist als 1,0 mm, bevorzugt geringer ist als 0,5 mm.

4. Gasaustauschmembranvorrichtung nach einem der Ansprüche 1 bis 3, **dadurch gekennzeichnet, dass** der mindestens eine Schlauch (3) eine Wandstärke der gesamten geschäumten Wandung (8) hat, die geringer ist, als 0,5 mm, bevorzugt geringer ist als 0,2 mm.

5. Gasaustauschmembranvorrichtung nach einem der Ansprüche 1 bis 4, **dadurch gekennzeichnet, dass** die Kavitäten (9) innerhalb des Schaums eine typische Größe haben zwischen 0,01 mm und 0,1 mm, bevorzugt zwischen 0,025 mm und 0,075 mm.

6. Verfahren zur Herstellung einer Gasaustauschmembranvorrichtung ; nach einem der Ansprüche 1 bis 5, mit folgenden Schritten:
- Extrudieren eines ein Treibmittel enthaltenden Ausgangsmaterials,
- Aufschäumen des Extrudats,
wobei ein schlauchförmiges Extrudat aus geschäumten, geschlossenporigen, vernetzten Silikon Kautschutz erzeugt wird, wobei aus dem Extrudat eine Mehrzahl von Schläuchen (3) abgelängt wird, die anschließend miteinander zu einem Bündel (2) angeordnet und fixiert werden.

7. Verfahren nach Anspruch 6, **dadurch gekennzeichnet, dass** das Fixieren der Schläuche (3) mit Hilfe eines Silikonklebers erfolgt

## Claims

1. Gas exchange membrane device for a medical application, in particular for use in an artificial lung, comprising a gas exchange membrane (3; 11) with an arrangement of foamed, closed-cell material, wherein the gas exchange membrane (3; 11) is designed such that a separation of the gas side from the blood side of the gas exchange membrane (3; 11) is possible such that a gas exchange may be performed through the gas exchange membrane (3; 11) and that an exchange of liquid and/or solid medium, in particular blood, blood plasma and/or contamination, through the gas exchange membrane (3; 11) is prevented, wherein the foamed material is formed in the shape of a bundle (2) of tubes (3), wherein the tubes (3) are secured relative to each other,
**characterized by**
an arrangement of the gas exchange membrane device of foamed, closed-cell, cross-linked silicone rubber.

2. Gas exchange membrane device according to claim 1, **characterized in that** the foamed material is platinum cross-linked or peroxide cross-linked.

3. Gas exchange membrane device according to claim 1 or 2, **characterized in that** the at least one tube (3) has an outer diameter which is smaller than 1.0 mm, preferably smaller than 0.5 mm.

4. Gas exchange membrane device according to one of claims 1 to 3, **characterized in that** the at least one tube (3) has a wall thickness of the entire foamed wall (8) which is smaller than 0.5 mm, preferably smaller than 0.2 mm.

5. Gas exchange membrane according to one of claims 1 to 4, **characterized in that** the cavities (9) in the foam have a typical size of between 0.01 mm and 0.1 mm, preferably between 0.025 mm and 0.075 mm.

6. Method for the production of a gas exchange membrane device according to one of claims 1 to 5, the method comprising the following steps:
- extruding a basic material containing a foaming agent;
- foaming the extrudate, wherein a tube-shaped extrudate is produced from foamed, closed-cell, cross-linked silicone rubber, with the extrudate being cut into a plurality of tubes (3) of a defined length which are then arranged in a bundle (2) and secured relative to each other.

7. Method according to claim 6, **characterized in that** securing takes place by means of a silicone glue.

## Revendications

1. Dispositif de membrane d'échange gazeux pour une utilisation médicale, notamment pour l'emploi dans un poumon artificiel, comprenant une membrane d'échange gazeux (3 ; 11) comportant une construction dans un matériau en mousse avec des pores fermées, la membrane d'échange gazeux (3 ; 11) étant conçue de telle manière qu'une séparation, entre un côté comprenant le gaz, et un côté comprenant le sang de la membrane d'échange gazeux (3 ; 11), est possible, de sorte qu'un échange par l'intermédiaire de la membrane d'échange gazeux (3 ; 11) puisse avoir lieu, et qu'un échange entre le milieu liquide et/ou solide, notamment du sang, du plasma sanguin et/ou des impuretés, puisse être empêché par la membrane d'échange gazeux (3 ; 11), le matériau en mousse étant conçu sous la forme d'un paquet (2) de tuyaux (3), les tuyaux (3) étant fixés les uns aux autres,
**caractérisé par**
une construction du dispositif de membrane d'échange gazeux dans un caoutchouc de silicone réticulé en mousse, avec des pores fermées.

2. Dispositif de membrane d'échange gazeux selon la revendication 1 **caractérisé en ce que** le matériau en mousse est réticulé au platine ou réticulé au peroxyde.

3. Dispositif de membrane d'échange gazeux selon les revendications 1 ou 2 **caractérisé en ce qu'**au moins l'un des tuyaux (3) possède un diamètre externe qui est inférieur à 1,0 mm, de préférence inférieur à 0,5 mm.

4. Dispositif de membrane d'échange gazeux selon l'une des revendications de 1 à 3 **caractérisé en ce qu'**au moins un tuyau (3) possède une épaisseur de paroi de la paroi globale en mousse (8) qui est inférieure à 0,5 mm, de préférence inférieure à 0,2 mm.

5. Dispositif de membrane d'échange gazeux selon l'une des revendications de 1 à 4 **caractérisé en ce que** les pores (9) à l'intérieur de la mousse possèdent une taille typique entre 0,01 mm et 0,1 mm, de préférence entre 0,025 mm et 0,075 mm.

6. Procédé de fabrication d'un dispositif de membrane d'échange gazeux selon l'une des revendications de 1 à 5 comprenant les étapes suivantes :
- extrusion d'un produit de départ contenant un produit provoquant une effervescence,
- formation de la mousse du produit d'extrusion,
un produit d'extrusion en forme de tuyau en caoutchouc de silicone réticulé en mousse avec des pores fermées étant réalisé, où une multitude de tuyaux (3) est formée à partir du produit d'extrusion, tuyaux qui sont ensuite disposés jointifs les uns aux autres pour former un paquet (2) et sont fixés.

7. Procédé selon la revendication 6 **caractérisé en ce que** la fixation des tuyaux (3) s'effectue à l'aide d'une colle silicone.
